(19) Europäisches Patentamt
European Patent Office
Office européen des brevets

(11) **EP 1 917 855 A1**

(12) **EUROPEAN PATENT APPLICATION**

(43) Date of publication:
**07.05.2008 Bulletin 2008/19**

(51) Int Cl.:
*A01N 37/06* (2006.01)    *A61K 8/19* (2006.01)
*A61K 8/36* (2006.01)    *A61Q 19/10* (2006.01)

(21) Application number: **07119021.9**

(22) Date of filing: **22.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **27.10.2006 JP 2006292518**

(71) Applicant: **MIURA CO., LTD.**
**Matsuyama-shi**
**Ehime 799-2696 (JP)**

(72) Inventors:
• **YOSHINARI, Yuji**
**Matsuyama-shi Ehime 799-2696 (JP)**

• **TAKAI, Masaki**
**Matsuyama-shi Ehime 799-2696 (JP)**
• **HATORI, Makoto**
**Matsuyama-shi Ehime 799-2696 (JP)**
• **OKAMOTO, Yuki**
**Matsuyama-shi Ehime 799-2696 (JP)**

(74) Representative: **Marchant, James Ian**
**Elkington and Fife LLP**
**Prospect House**
**8 Pembroke Road**
**Sevenoaks, Kent TN13 1XR (GB)**

(54) **Skin Disinfectant**

(57) The skin disinfectant, which is able to sterilize skin by the action of a surfactant without damaging skin health conditions, contains water, from which polyvalent cations are removed and to which a sodium ion is added, and a surfactant. The surfactant is, for example, a fatty acid salt. When the skin disinfectant is applied onto skin, fungi and bacteria adhered or parasitic onto skin is sterilized by the action of the surfactant. At this time, the surfactant is not likely to give an irritation to skin due to the action of the water, from which polyvalent cations are removed and to which a sodium ion is added, and thus the skin health conditions tend not to be damaged. Accordingly, the skin disinfectant is useful as a remedy for, for example, a ringworm infectious disease.

EP 1 917 855 A1

**Description**

Technical Field

The present invention relates to a skin disinfectant, in particular, a skin disinfectant using a surfactant.

Background Art

Skin sometimes develops illnesses by infection or parasitism of fungi and bacteria. For example, skin where ringworm, a family of mold, is parasitic develops a ringworm infectious disease (so called athlete's foot). Various medical agents are provided in order to improve symptoms of such skin diseases, but any medicines contain chemical components with a strong sterilizing action. Thereby, side effects caused by the chemical components are concerned.
On the other hand, it is known that surfactants such as soap have a sterilizing action, and thus skin disinfectants using such surfactants have been proposed. When a skin disinfectant of this kind is applied onto skin where a ringworm infectious disease or the like develops, the surfactant sterilizes the fungi or bacteria, and thereby the symptom of the disease improves. However, the surfactant may give irritation to the skin at the same time, and the skin becomes dry and develops itchy feeling or scale in some cases. Therefore, a skin disinfectant, which uses a surfactant, but reforms irritation to skin, has been proposed. For example, Japanese Unexamined Patent Publication (Kohyo) No.10-500962 (JP 1998-500962 A) discloses a low irritating antifungal/antibacterial liquid cleaning composition, which contains a surfactant that is less irritating than soap is, a buffer solution such as phosphoric acid and carbonic acid, and water.
However, since such an antifungal/antibacterial liquid cleaning composition needs to use a surfactant that is less irritating than soap, selection of a surfactant is limited. There are even such cases that a required sterilizing action cannot be expected unless an antifungal/antibacterial agent is used in combination.
An object of the present invention is to realize a disinfection treatment of skin by the action of a surfactant without damaging skin health conditions.
The term "skin" is the concept including mucous and cornea in the present application.

Summary of the Invention

A skin disinfectant of the present invention contains water, from which polyvalent cations are removed and to which a sodium ion is added, and a surfactant.
When the skin disinfectant is applied to skin, fungi and bacteria adhered or parasitic onto the skin are sterilized by the action of the surfactant. At this time, the surfactant is not likely to give an irritation to the skin nor damage skin health conditions due to the action of water, from which polyvalent ions are removed and to which a sodium ion is added. That is, the skin disinfectant of the present invention enables a disinfection treatment of skin without damaging skin health conditions.
Accordingly, the skin disinfectant effectively works, for example, as a remedy for a ringworm infectious disease.
The surfactant used in the skin disinfectant of the present invention is, for example, a fatty acid salt. Particularly, unsaturated fatty acid salts are preferable. With regard to the unsaturated fatty acid salt, for example, at least one selected from the group consisting of linoleic acid, linolenic acid, myristoleic acid and palmitoleic acid is used.
Other objects and effects of the present invention will be described in detail hereinafter.

Brief Description of the Drawings

Fig. 1 is a graph showing evaluation results of Examples 1 and 2 and Comparative Example 1 and 2.
Fig. 2 is a graph showing results of Examples 3 to 11.
Fig. 3 is a graph showing results of Examples 12 and 13.
Fig. 4 is a graph showing results of Examples 14 and 15.
Fig. 5 is a graph showing results of Examples 16 and 17.
Fig. 6 is a graph showing results of Examples 18 and 19 and Comparative Examples 3 and 4.
Fig. 7 is a graph showing a result of stratum corneum moisture content measured in Evaluation A of Example 21.
Fig. 8 is a graph showing a result of skin elasticity measured in Evaluation A of Example 21.
Fig. 9 is a graph showing a result of texture density measured in Evaluation A of Example 21.

**EP 1 917 855 A1**

Description of the Preferred Embodiment

The skin disinfectant of the present invention contains water from which polyvalent cations are removed and to which a sodium ion is added (hereafter such water is called "functional water" in some cases), and a surfactant.

The functional water used in the present invention is obtained by treatment of water (raw water), such as tap, ground, river, lake and well water, with a cation exchange resin. In this treatment, a calcium ion (bivalent cation), magnesium ion (bivalent cation), copper ion (bivalent cation), iron ion (bivalent and trivalent cations), aluminum ion (trivalent cation) and the like contained in the raw water are exchanged with a sodium ion (monovalent cation) contained in the cation exchange resin.

The cation exchange resin used for the treatment of raw water is a synthetic resin, wherein a suflonic acid group is introduced to a matrix of a cross-linked three dimensional polymer such as a copolymer of styrene and divinylbenzene, and the sulfonic acid group forms a sodium salt.

In the functional water, it is preferable that a concentration of polyvalent cations is commonly adjusted to less than 0.2 mmol/l, and particularly preferable to be adjusted to less than the measurement limit, which signifies substantially zero level. Here, the concentration of polyvalent cations denotes a concentration measured on the basis of ICP emission spectroscopic analysis.

On the other hand, in the functional water, it is preferable that a concentration of sodium ions is commonly adjusted to 0.3 mmol/l or more and less than 500 mmol/l, and more preferable to be adjusted to 0.5 mmol/l or more and less than 200 mmol/l. Here, the concentration of a sodium ion denotes a concentration measured on the basis of ICP emission spectroscopic analysis.

A surfactant used in the present invention is not particularly limited. Examples thereof include anionic, cationic, ampholytic and nonionic surfactants.

Examples of the anionic surfactant include fatty acid salts (soaps), alkylbenzene sulfonate salts, alkyl sulfate salts, $\alpha$-olefin sulfonate salts and N-acyl glutamate salts. Two or more of these anionic surfactants may be used in combination.

Examples of the cationic surfactant include N-alkyltrimethyl ammonium chloride and N-alkylbenzyl dimethyl ammonium chloride. Two or more of these cationic surfactants may be used in combination.

Examples of the ampholytic surfactant include N-alkyl-$\beta$-alanine and N-alkylcarboxy betaine. Two or more of these ampholytic surfactants may be used in combination.

Examples of the nonionic surfactant include polyoxyethylene alkyl ether, polyoxyethylene alkylphenyl ether, fatty acid diethanol amide and fatty acid sucrose ester. Two or more of these nonionic surfactants may be used in combination.

In the present invention, the above various surfactants can be used in combination with other kinds of surfactants.

Preferred surfactants used in the present invention are fatty acid salts, particularly alkali metal salts of saturated or unsaturated fatty acid having 5 to 22 carbon atoms. An alkali metal salt of saturated fatty acid and an alkali metal salt of unsaturated fatty acid can be used in combination.

With regard to the saturated fatty acid salts, those having 12 to 16 carbon atoms are particularly preferred. Specifically, sodium salts and potassium salts of lauric acid, myristic acid, pentadecylic acid and palmitic acid are exemplified. On the other hand, with regard to the unsaturated fatty acid salts, those having 14 to 18 carbon atoms are preferred, and those having a larger number of unsaturated bonds between carbons are particularly preferred. Specific examples of the unsaturated fatty acid include sodium salts and potassium salts of myristoleic acid, palmitoleic acid, oleic acid, linoleic acid and linolenic acid.

As for fatty acid salts, it is preferable to use unsaturated fatty acid salts since sterilizing ability of the skin disinfectant of the invention can be enhanced. Particularly, salts of linoleic acid, linolenic acid, myristoleic acid and palmitoleic acid are preferred, and sodium salts thereof are more preferred.

In the skin disinfectant of the present invention, an amount of a surfactant to be used is preferably adjusted to from 10 mg to 400 g per liter of the functional water, and more preferably adjusted to from 100 mg to 200 g. When the amount of the surfactant is less than 10 mg, there is a possibility that the disinfectant of the present invention does not exhibit effective sterilization action. On the contrary, when it exceeds 400 g, the surfactant is apt to remain on skin, and there is a possibility that fungi and bacteria rather propagate in the skin easily by consuming the residual surfactant as a source of nutrient.

The skin disinfectant of the present invention may contain some other components other than the above functional water and surfactant to the extent that they do not spoil the objects of the present invention. Examples of other components include fragrant materials such as a grapefruit oil, spearmint oil, nutmeg oil and mandarin oil, and antioxidants such as tocopherol, ascorbyl stearate ester, sodium erythorbate, ascorbic acid, citric acid and dibutyl hydroxytoluene. Two or more of the fragrant materials and antioxidants may be used in combination.

The skin disinfectant of the present invention is easily prepared through the processes of treating raw water with the above cation exchange resin, and to the resulting functional water, properly adding a surfactant and, if necessary, the above other components. Accordingly, the skin disinfectant can be easily produced in large quantity, and also can be produced at low cost.

Skin to which the skin disinfectant of the present invention can be applied includes entire body skin including mucous and cornea, and is not particularly limited. Although an application method to skin is not particularly limited, it is preferable to employ a method that enables the skin to be fully moistened with the skin disinfectant, commonly such as spreading or spraying it over the skin. The skin to which the skin disinfectant is applied is preferably washed with water after applying thereof, and then is wiped out to remove water and dried, in general. At this time, it is preferable to use the above functional water as the water to wash the skin disinfectant off.

The skin disinfectant of the present invention, which is applied to skin, is able to effectively sterilize fungi and bacteria adhered or parasitic onto skin by the action of a surfactant. At this time, the skin disinfectant can alleviate, by the action of the functional water, that the surfactant provides irritation to skin so that dryness of the skin is suppressed and the skin health conditions are hardly damaged.

In addition, the skin over which the skin disinfectant of the present invention is applied tends not to retain a surfactant, which may otherwise be a source of nutrient for fungi and bacteria, due to the action of the functional water. Particularly, when the skin is washed with the functional water after applying the skin disinfectant, a surfactant hardly remains on a surface of the skin. Consequently, the skin over which the skin disinfectant is applied turns into an environment where fungi and bacteria hardly propagate, and thus the diseases resulting from fungi and bacteria, such as a ringworm infectious disease, is readily cured.

The above effects tend to be achieved more easily when the skin disinfectant of the present invention is applied to skin continuously.

In the above embodiment, water from which polyvalent cations are removed and to which a sodium ion is added is used as functional water, but the functional water may be such that polyvalent cations are removed and an alkali metal ion other than a sodium ion, such as a potassium ion, is added. Functional water of this kind can be obtained by treating raw water with a cation exchange resin, wherein a sulfonic acid group forms an alkali metal salt such as a potassium salt.

Examples

Example 1

A soap (trade name of "Nantaro Soap" manufactured by Miura Co., Ltd.) was dissolved in water, thereby soapy water (skin disinfectant) of 0.01% by weight concentration was prepared. The water used here was functional water obtained by treating tap water supplied in Matsuyama City, Ehime Japan, with a cation exchange resin. The water satisfied the following conditions: a concentration of polyvalent cation is less than 0.2 mmol/l; and a concentration of sodium ions is 0.3 mmol/l or more and less than 500 mmol/l.

Example 2

Soapy water (skin disinfectant) was prepared in the same manner as in Example 1, except for changing the concentration to 0.05% by weight.

Comparative Example 1

A soap (trade name of "Nantaro Soap" manufactured by Miura Co., Ltd.) was dissolved in tap water supplied in Matsuyama City, Ehime Japan, thereby soapy water having a concentration of 0.01% by weight was prepared.

Comparative Example 2

Soapy water was prepared in the same manner as in Comparative Example 1, except for changing the concentration to 0.05% by weight.

Evaluation of Examples 1 and 2 and Comparative Examples 1 and 2

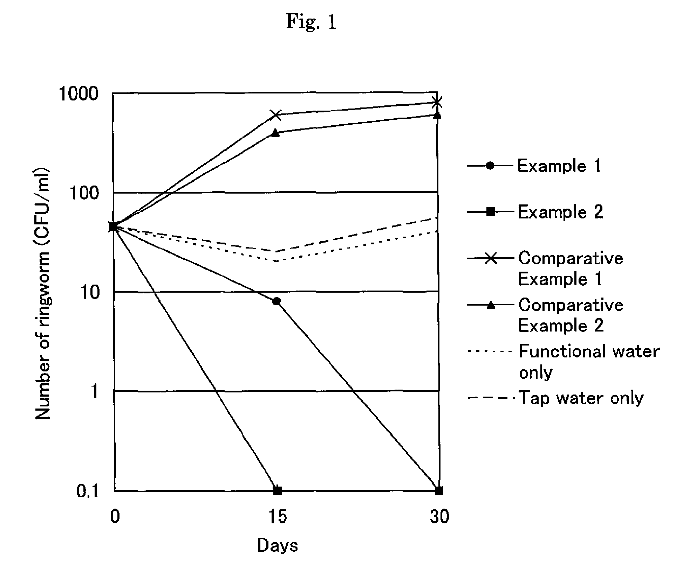

Ringworm (Trichophyton rubrum NBRC32409) was added to the soapy water prepared in Examples 1 and 2 and Comparative Examples 1 and 2 in an amount of about 30 CFU/ml (the unit "CFU" stands for "colony forming unit"), which was allowed to stand at a temperature of 25°C for 30 days. During this time, the diachronic change in the number of ringworm in the soapy water was measured everyday. The results are shown in Fig. 1. For reference, Fig. 1 also shows diachronic changes in the number of ringworm in case of adding ringworm to functional water alone that was used in Examples 1 and 2 (denoted as "functional water only" in Fig. 1), and in case of adding ringworm to tap water alone that was used in Comparative Examples 1 and 2 (denoted as "tap water only" in Fig. 1). The measurement of the number of ringworm was conducted as follows.

Soapy water (50 ml) containing ringworm in a 100 ml Erlenmeyer flask was stirred at a rate of 10, 000 rpm for 5 minutes using a homogenizer (trade name of "Ace Homogenizer AM-3" manufactured by Nihonseiki Kaisha LTD.) to dissociate the ringworm, and then the soapy water was subjected to ultrasonic waves. A sample of 100 $\mu$l was taken out from the soapy water and used without being diluted. The sample was cultured at 25°C for 5 days using a PDA (Potato Dextrose Agar) plate medium containing chloramphenicol, and the number of the growing ringworm colonies was counted by visual observation. Based on the result, the number of ringworm contained in the soapy water was calculated using the following equation (1)

Number of ringworm (CFU/ml) =

Number of ringworm colonies × 10          (1)

According to Fig. 1, the soapy water used in Examples 1 and 2 excels in sterilizing effect against ringworm.

Examples 3 to 11

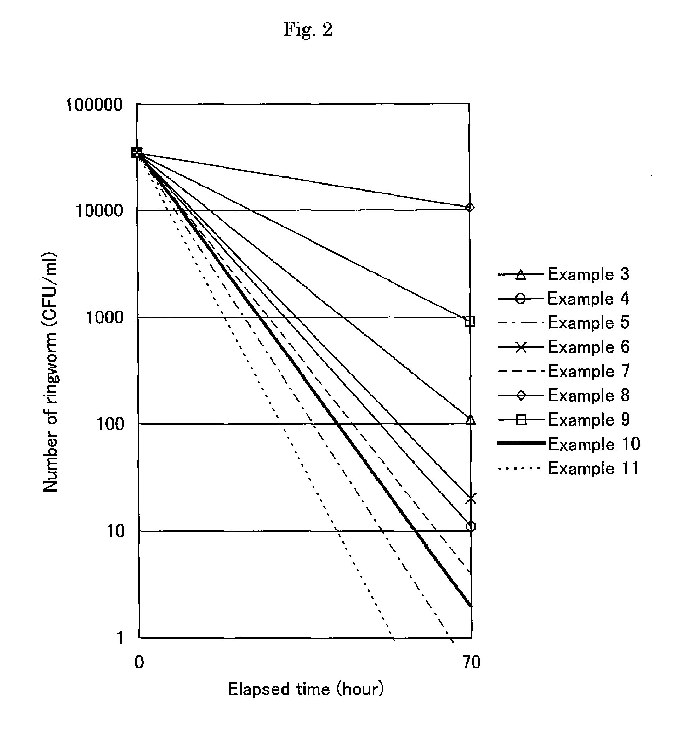

A fatty acid sodium salt shown in Table 1 was dissolved in functional water, which was obtained by treating tap water supplied in Matsuyama City, Ehime Japan, with a cation exchange resin, to prepare a 5 mM aqueous solution of fatty acid sodium salt (skin disinfectant). Ringworm (Trichophyton mentagrophytes) was added to the aqueous solution of fatty acid sodium salt in an amount of about $2 \times 10^4$ CFU/ml, which was shaken at 35°C, and then the diachronic change in the number of the ringworm was measured over the following 70 hours. The number of the ringworm was measured as follows. After shaking the aqueous solution of fatty acid sodium salt containing ringworm, a sample of 100 $\mu$l was taken therefrom and properly diluted. The diluted sample was cultured at 25°C for 5 days using a PDA (Potato Dextrose Agar) plate medium containing chloramphenicol, and the number of the growing ringworm colonies was counted by visual observation. Based on the result, the number of the ringworm contained in the skin disinfectant was calculated using the following equation (2). The results are shown in Fig. 2.

Number of ringworm (CFU/ml) =

Number of ringworm colonies × Dilution rate × 10

(2)

Table 1

| Examples | Fatty acid sodium salt | | |
| --- | --- | --- | --- |
| | Name | Number of carbon atom | Number of carbon-carbon double bond |
| 3 | Sodium laurate | 12 | 0 |
| 4 | Sodium myristate | 14 | 0 |
| 5 | Sodium myristolate | 14 | 1 |
| 6 | Sodium palmitate | 16 | 0 |
| 7 | Sodium palmitolate | 16 | 1 |
| 8 | Sodium stearate | 18 | 0 |
| 9 | Sodium oleate | 18 | 1 |
| 10 | Sodium linoleate | 18 | 2 |

(continued)

| Examples | Fatty acid sodium salt | | |
| | Name | Number of carbon atom | Number of carbon-carbon double bond |
|---|---|---|---|
| 11 | Sodium linolenate | 18 | 3 |

According to Fig. 2, it is found that an aqueous solution of a fatty acid sodium salt exhibits high sterilizing ability particularly when a fatty acid sodium salt having 12 to 16 carbon atoms is used. When carbon numbers of the fatty acid sodium salts are identical, unsaturated fatty acid sodium salts, particularly those having many carbon-carbon double bonds, exhibit higher sterilizing power.

Example 12

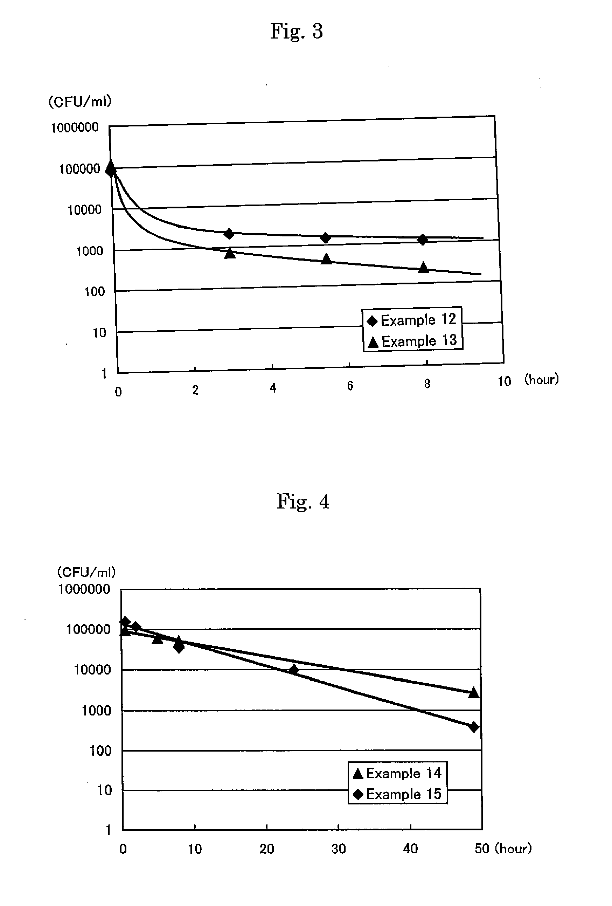

A sodium myristate salt was dissolved in functional water, which was obtained by treating tap water supplied in Matsuyama City, Ehime Japan, with a cation exchange resin, to prepare a skin disinfectant having a concentration of 5 mM. Black mold (Cladosporium sphaerospermum NBRC4460) was added to the skin disinfectant in an amount of about $1 \times 10^5$ CFU/ml, which was shaken at 35°C, and then the diachronic change in the number of the black mold spores was measured. The measurement of the number of the black mold spores was conducted as follows. First, the skin disinfectant containing the black mold was properly diluted with a sterilized phosphate buffer solution. Then, a sample of 100 $\mu$l taken therefrom was cultured at 25°C for 5 days using a PDA (Potato Dextrose Agar) plate medium containing chloramphenicol, and the number of the growing black mold colonies was counted by visual observation. Based on the result, the number of the black mold contained in the skin disinfectant was calculated using the following equation (3). The results are shown in Fig. 3.

```
Number of black mold spores (CFU/ml) =

     Number of black mold colonies × Dilution rate × 10
```

$$\tag{3}$$

Example 13

The operation was implemented in the same manner as in Example 12, except for using a sodium linolenate salt instead of a sodium myristate salt, and the diachronic change in the number of black mold spores was measured. The results are shown in Fig. 3.

Example 14

A sodium myristate salt was dissolved in functional water, which was obtained by treating tap water supplied in Matsuyama City, Ehime Japan, with a cation exchange resin, to prepare a skin disinfectant having a concentration of 5 mM. Colon bacillus (E. coli NBRC3301) was added to the skin disinfectant in an amount of about $1 \times 10^5$ CFU/ml, which was shaken at 35°C, and then the diachronic change in the number of the colon bacillus was measured. The number of the colon bacillus was measured as follows. First, the skin disinfectant containing the colon bacillus was properly diluted with a sterilized phosphate buffer solution. Then, a sample of 100 $\mu$l taken therefrom was cultured at 35°C for 3 days using a standard agar medium, and the number of the growing colon bacillus colonies was counted by visual observation. Based on the result, the number of the colon bacillus contained in the skin disinfectant was calculated using the following equation (4). The results are shown in Fig. 4.

```
Number of colon bacillus (CFU/ml) =

    Number of colon bacillus colonies × Dilution rate × 10
```

$$(4)$$

Example 15

The operation was implemented in the same manner as in Example 14, except for using a sodium linolenate salt instead of a sodium myristate salt, and the diachronic change in the number of colon bacillus was measured. The results are shown in Fig. 4.

Example 16

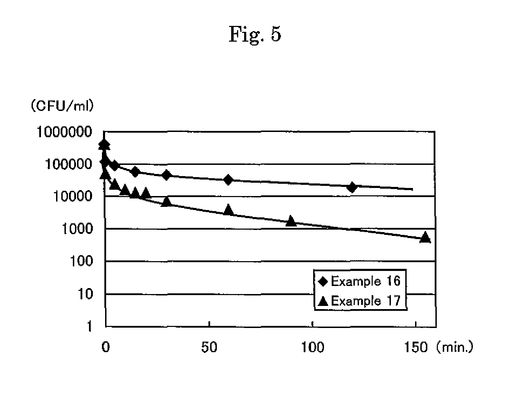

A sodium myristate salt was dissolved in functional water, which was obtained by treating tap water supplied in Matsuyama City, Ehime Japan, with a cation exchange resin, to prepare a skin disinfectant having a concentration of 5 mM. Staphylococcus aureus bacteria (S. aureus NBRC13276) was added to the skin disinfectant in an amount of about $1 \times 10^5$ CFU/ml, which was shaken at 35°C, and then the diachronic change in the number of the staphylococcus aureus bacteria was measured. The number of the staphylococcus aureus bacteria was measured as follows. First, the skin disinfectant containing the staphylococcus aureus bacteria was properly diluted with a sterilized phosphate buffer solution. Then, a sample of 100 $\mu$l taken therefrom was cultured at 35°C for 3 days using a standard agar medium, and the number of the growing staphylococcus aureus bacterial colonies was counted by visual observation. Based on the result, the number of the staphylococcus aureus bacteria (S. aureus bacteria) contained in the skin disinfectant was calculated using the following equation (5). The results are shown in Fig. 5.

```
Number of S. aureus bacteria (CFU/ml) =

   Number of S. aureus bacterial colonies × Dilution rate × 10
```

$$(5)$$

Example 17

The operation was implemented in the same manner as in Example 16, except for using a sodium linolenate salt instead of a sodium myristate salt, and the diachronic change in the number of staphylococcus aureus bacteria was measured. The results are shown in Fig. 5.

Example 18

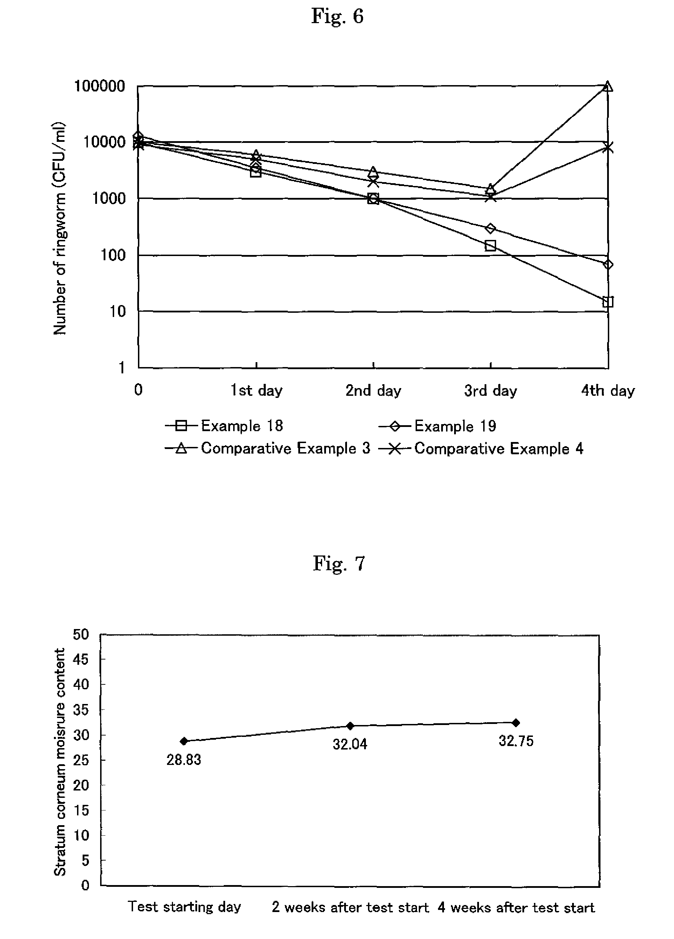

A sodium linoleate salt was dissolved in functional water, which was obtained by treating tap water supplied in Matsuyama City, Ehime Japan, with a cation exchange resin, to prepare a skin disinfectant having a concentration of 1 mM. Ringworm (Trichophyton mentagrophytes) was added to the skin disinfectant in an amount of about $1 \times 10^4$ CFU/ml, which was shaken at 35°C, and then the diachronic change in the number of the ringworm was measured. The number of the ringworm was measured in the same manner as in Examples 3 to 11. The results are shown in Fig. 6.

Example 19

The operation was implemented in the same manner as in Example 18, except for using a sodium linolenate salt instead of a sodium linoleate salt, and the diachronic change in the number of ringworm was measured. The results are shown in Fig. 6.

Comparative Example 3

The operation was implemented in the same manner as in Example 18, except for using tap water of Matsuyama City, Ehime Japan, instead of functional water, and the diachronic change in the number of ringworm was measured. The results are shown in Fig. 6.

Comparative Example 4

The operation was implemented in the same manner as in Example 19, except for using tap water of Matsuyama City, Ehime Japan, instead of functional water, and the diachronic change in the number of ringworm was measured. The results are shown in Fig. 6.

Example 20

Potassium linoleate (prepared by saponification of 2 ml of linoleic acid manufactured by Wako Pure Chemicals Industries with potassium hydroxide) was dissolved in functional water, which was obtained by treating tap water supplied in Matsuyama City, Ehime Japan, with a cation exchange ion and also satisfied the conditions that a concentration of polyvalent cations was less than 0.2 mmol/l and a concentration of sodium ions was 0.3 mmol/l or more and less than 500 mmol/l, to prepare a total amount of 20 ml of a skin disinfectant. Then, using the skin disinfectant, 5 examinees whose resident microbiota in their hands and fingers had measured in advance washed their hands. Hand-washing was performed according to an everyday hand-washing method recommended by the Japan Food Hygiene Association, and the above functional water was used as rinsing water. One hour after the hand-washing, the number of resident microbiota in their hand and fingers were measured. The result of the examination on a reduction rate of resident microbiota is shown in Table 2. The result shown in Table 2 is a mean value of 5 examinees. Resident microbiota in hands and fingers was measured by collecting the resident microbiota by a glove juice method.

Comparative Examples 5

A skin disinfectant of the same kind as that of Example 20 was prepared using tap water of Matsuyama City, Ehime Japan, instead of the functional water. Using the skin disinfectant, the same 5 examinees as in Example 20 washed their hands in the same manner as in Example 20 (except for using tap water of Matsuyama City, Ehime Japan, as rinsing water), and a reduction rate in the number of resident microbiota was examined. The result is shown in Table 2. The result shown in Table 2 is a mean value of 5 examinees.

Table 2

|  | Reduction rate of resident microbiota (%) |
| --- | --- |
| Example 20 | 60.9 |
| Comparative Example 5 | 12.0 |

Example 21

The skin conditions were studied on 8 examinees who took a bath everyday for 4 weeks using the skin disinfectant. The examinees were female aging from 30 to 47 years old (average age: 36.9 years old). The skin disinfectants used herein by respective examinees were prepared by dissolving cleaning agents shown in Table 3 in the functional water. The functional water was obtained by treating tap water with a cation exchange resin, and satisfied the conditions that a concentration of polyvalent cation is less than 0.2 mmol/l and a concentration of sodium ions is 0.3 mmol/l or more and less than 500 mmol/l.

Table 3

| Examinee | | Cleaning agent | | |
| --- | --- | --- | --- | --- |
| Number | Age | Kind | Name of manufacturer | Trade name |
| 1 | 42 | Body shampoo | Unilever Japan | Dove body wash |
| 2 | 37 | Body shampoo | Shiseido Co., Ltd. | Kuyura |

(continued)

| Examinee | | Cleaning agent | | |
|---|---|---|---|---|
| Number | Age | Kind | Name of manufacturer | Trade name |
| 3 | 36 | Body shampoo | Kanebo Cosmetic Inc. | Naive body soap N, moisture milk |
| 4 | 47 | Body shampoo | Amway Japan Ltd. | Satinique body shampoo |
| 5 | 34 | Soap | Kanebo Cosmetic Inc. | Silk soap |
| 6 | 30 | Body shampoo | Unilever Japan | Dove body care wash |
| 7 | 30 | Body shampoo | Kao Corp. | Biore |
| 8 | 39 | Soap | Yugen Kaisha Neba Juku | Soap Seseragi |

While taking a bath, the examinees washed their bodies with the skin disinfectant, rinsed the skin disinfectant off with the above functional water alone, and soaked in a bathtub filled with the heated functional water alone.

<Evaluation A>

Each item of stratum corneum moisture content, skin elasticity and texture density was measured on every examinee by the following methods. The mean value of each item was compared among the day when the test was started, 2 weeks after the test start and 4 weeks after the test start.

(Stratum corneum moisture content)

An electric characteristic of skin, that is, capacitance of skin surface was measured to determine stratum corneum moisture content with a measuring device of stratum corneum moisture content (trade name of "Corneometer CM825" manufactured by Courage + Khazaka Electronic GmbH). The measurement was conducted 3 times, and the mean value was served as the measurement value. The results are shown in Fig.7.

(Skin elasticity)

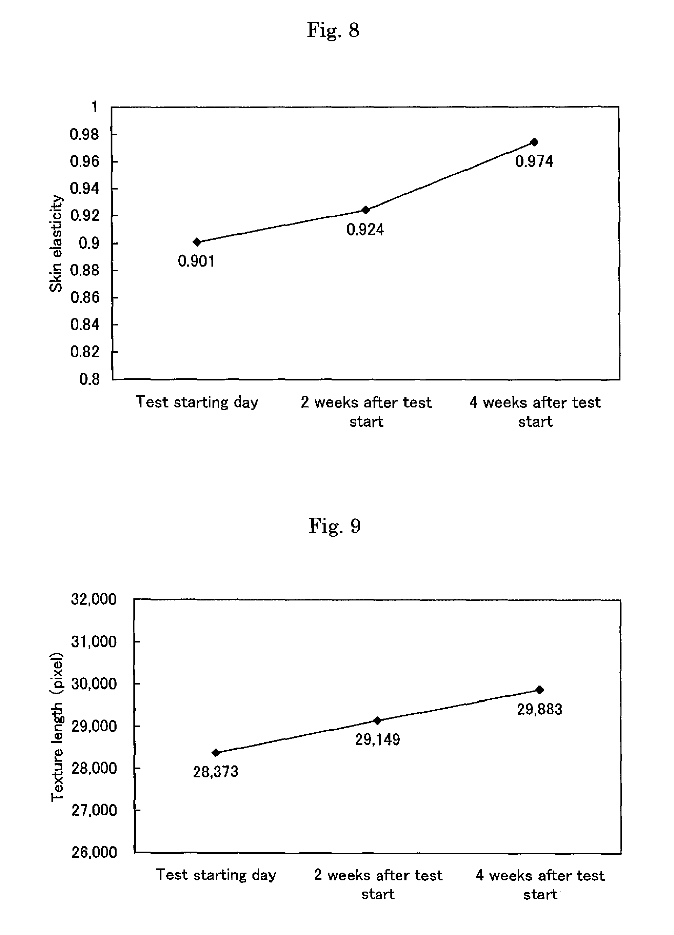

Skin electricity was measured using a measuring device of skin elasticity (trade name of "Cutometer SEM575" manufactured by Courage + Khazaka Electronic GmbH), which is equipped with a measurement probe having a suction hole, that is, a negative pressure aspirator, a pressure sensor, and a computer for operation thereof and data processing. In this measurement, when the probe is guided to a skin surface to start the measurement, a negative pressure is applied to the probe suction hole and thus the skin inside the suction hole is sucked in. Then, a height of the sucked skin was measured with a light sensor contactlessly without generation of friction or mechanical action. The measurement was conducted 4 times, and the mean value was served as the measurement value. The results are shown in Fig. 8.

(Texture density)

Texture density was measured by applying image analysis software (trade name of "Skin Analysis Software" manufactured by Inforward Inc.) for the purpose of evaluating a state of the texture distribution to a color image of skin obtained by a device with a trade name of "Microscope VI-27" manufactured of FineOpt Co., Ltd. Specifically, image data processing was applied by the above image analysis software to a color image obtained so as to emphasize and extract the texture region, and a total length of the texture region (unit: pixel) was calculated. The results are shown in Fig. 9. Texture density denotes a percentage (%) of a texture length in an area of the obtained color image, and is therefore substantially in a relation of "texture length (pixel)" = "texture density (%)".

<Evaluation B>

On the same day when each item in Evaluation A was measured, drying condition of skin and existence or nonexistence of scale were evaluated on every examinee, according to a diagnosis by a doctor. The results are shown in Table 4. In Table 4, evaluation criteria on each item are as follows.

(Drying condition)

None: The symptom is not observed.
Minor: The symptom is slightly observed.
Mild: The symptom is somewhat observed.
Moderate: The symptom is clearly observed.
Severe: The symptom is remarkably observed.

(Existence or nonexistence of scale)

None: The symptom is not observed.
Minor: The symptom is slightly observed.
Mild: The symptom is somewhat observed.
Moderate: The symptom is clearly observed.
Severe: The symptom is remarkably observed.

Table 4

|  |  | None | Minor | Mild | Moderate | Severe |
|---|---|---|---|---|---|---|
| Drying condition | Test starting day | 0 | 1 | 5 | 2 | 0 |
|  | Two weeks after test start | 0 | 5 | 3 | 0 | 0 |
|  | Four weeks after test start | 0 | 7 | 1 | 0 | 0 |
| Existence or nonexistence of scale | Test starting day | 1 | 6 | 1 | 0 | 0 |
|  | Two weeks after test start | 1 | 7 | 0 | 0 | 0 |
|  | Four weeks after test start | 7 | 0 | 1 | 0 | 0 |

<Evaluation C>

On the same day when each item in Evaluation A was measured, itchy feeling of skin based on the examinees' own complaint was evaluated according to a diagnosis by a doctor. The results are shown in Table 5. In Table 5, evaluation criteria on itchy feeling are as follows.

None: The symptom is not observed.
Minor: The symptom is slightly observed.
Mild: The symptom is somewhat observed.
Moderate: The symptom is clearly observed.
Severe: The symptom is remarkably observed.

Table 5

|  |  | None | Minor | Mild | Moderate | Severe |
|---|---|---|---|---|---|---|
| Itchy feeling | Test starting day | 3 | 1 | 3 | 1 | 0 |
|  | Two weeks after test start | 6 | 2 | 0 | 0 | 0 |
|  | Four weeks after test start | 8 | 0 | 0 | 0 | 0 |

<Evaluation D>

The results of a questionnaire relating to self-evaluation on the examinees' own skin are shown in Table 6, which was implemented to each examinee before and after the test.

Table 6

| | | Feel | Somewhat feel | Normal | Not quite feel | Not feel |
|---|---|---|---|---|---|---|
| There is a taut feeling after taking a bath | Before test start | 3 | 2 | - | 1 | 2 |
| | After test termination | 0 | 0 | - | 4 | 4 |
| Skin is dry | Before test start | 4 | 2 | 2 | 0 | 0 |
| | After test termination | 1 | 3 | 1 | 2 | 1 |
| Skin is smooth | Before test start | 0 | 3 | 2 | 1 | 2 |
| | After test termination | 2 | 4 | 1 | 0 | 1 |
| Skin has Skin has tightness and elasticity | Before test start | 0 | 0 | 5 | 1 | 2 |
| | After test termination | 0 | 3 | 4 | 0 | 1 |
| Skin texture is finer | Before test start | 0 | 2 | 4 | 1 | 1 |
| | After test termination | 0 | 5 | 2 | 1 | 0 |

The results of Evaluations A to D indicate that in case of taking a bath continuously using the skin disinfectant, skin health condition is hardly damaged, and rather it tends to improve.

The present invention can be practiced in other various forms without departing from the spirit and principal features thereof. In view of this, the embodiments or examples described above merely serve as exemplification in every respect and should not be construed restrictively. A scope of the present invention is defined by claims, and is by no means bound by the text of the specification. Furthermore, all modifications and alternations belonging to the equivalent scope of the claims fall within the scope of the present invention.

**Claims**

1.  A skin disinfectant comprising,
    water from which polyvalent cations are removed and to which a sodium ion is added, and
    a surfactant.

2.  The skin disinfectant according to claim 1, which is used for a ringworm infectious disease.

3.  The skin disinfectant according to claim 1 or 2, wherein the surfactant is a fatty acid salt.

4.  The skin disinfectant according to claim 3, wherein the fatty acid salt is an unsaturated fatty acid salt.

5.  The skin disinfectant according to claim 4, wherein the unsaturated fatty acid salt is at least one selected from the group consisting of linoleate, linolenate, myristolate and palmitolate.

Fig. 1

Fig. 2

Fig. 3

Fig. 4

Fig. 5

Fig. 6

Fig. 7

Fig. 8

Fig. 9

**European Patent Office**

**EUROPEAN SEARCH REPORT**

Application Number

EP 07 11 9021

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 486 558 A (KAO CORP [JP]) 15 December 2004 (2004-12-15) * paragraph [0008] - paragraph [0014]; claims; example 8 * ----- | 1-3 | INV. A01N37/06 A61K8/19 A61K8/36 A61Q19/10 |
| X | EP 0 507 004 A (REWO CHEMISCHE WERKE GMBH [DE] WITCO SURFACTANTS GMBH [DE]) 7 October 1992 (1992-10-07) * claims; examples * ----- | 1,2 | |
| X | US 2003/152533 A1 (TANG DIANA [US]) 14 August 2003 (2003-08-14) * examples shampoo,body,wash * ----- | 1,2 | |
| X | EP 1 479 365 A (UNILEVER PLC [GB]; UNILEVER NV [NL]) 24 November 2004 (2004-11-24) * claims; examples * ----- | 1,2 | |
| D,A | WO 95/32705 A (UNILEVER PLC [GB]; UNILEVER NV [NL]) 7 December 1995 (1995-12-07) * the whole document * ----- | 1 | TECHNICAL FIELDS SEARCHED (IPC) A01N A61K A61Q |

The present search report has been drawn up for all claims

| Place of search | Date of completion of the search | Examiner |
|---|---|---|
| Munich | 19 March 2008 | Donovan-Beermann, T |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 07 11 9021

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2008

| Patent document cited in search report | | Publication date | Patent family member(s) | | Publication date |
|---|---|---|---|---|---|
| EP 1486558 | A | 15-12-2004 | CN 1572291 A<br>DE 602004000627 T2<br>US 2005020461 A1 | | 02-02-2005<br>11-01-2007<br>27-01-2005 |
| EP 0507004 | A | 07-10-1992 | AT 121286 T<br>CA 2064482 A1<br>DE 4110852 A1<br>DK 507004 T3<br>ES 2073106 T3<br>GR 3015961 T3 | | 15-05-1995<br>05-10-1992<br>08-10-1992<br>21-08-1995<br>01-08-1995<br>31-07-1995 |
| US 2003152533 | A1 | 14-08-2003 | US 2004131573 A1 | | 08-07-2004 |
| EP 1479365 | A | 24-11-2004 | JP 2004346061 A<br>US 2004234558 A1 | | 09-12-2004<br>25-11-2004 |
| WO 9532705 | A | 07-12-1995 | AU 2735595 A<br>BR 9507819 A<br>CA 2186011 A1<br>CN 1148803 A<br>CZ 9603500 A3<br>DE 69532888 D1<br>DE 69532888 T2<br>EP 0762868 A1<br>ES 2218547 T3<br>HU 76537 A2<br>JP 10500962 T<br>PL 317427 A1<br>SK 152596 A3<br>US 5681802 A<br>US 5914300 A<br>ZA 9504286 A | | 21-12-1995<br>16-09-1997<br>07-12-1995<br>30-04-1997<br>14-05-1997<br>19-05-2004<br>28-04-2005<br>19-03-1997<br>16-11-2004<br>29-09-1997<br>27-01-1998<br>14-04-1997<br>04-06-1997<br>28-10-1997<br>22-06-1999<br>25-11-1996 |

EPO FORM P0459

For more details about this annex : see Official Journal of the European Patent Office, No. 12/82

**EP 1 917 855 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10500962 A